(19) Europäisches Patentamt
European Patent Office
Office européen des brevets

(11) Veröffentlichungsnummer : **0 104 473**
**B1**

(12) # EUROPÄISCHE PATENTSCHRIFT

(45) Veröffentlichungstag der Patentschrift : **22.01.86**

(21) Anmeldenummer : **83108520.4**

(22) Anmeldetag : **30.08.83**

(51) Int. Cl.⁴ : **C 07 C147/06**, C 07 C147/14, C 07 C149/40, A 01 N 41/10, A 01 N 31/16

(54) **Neue Phenoxyalkansäureverbindungen zur Regulierung des Pflanzenwachstums.**

(30) Priorität : **02.09.82 DE 3232624**

(43) Veröffentlichungstag der Anmeldung : **04.04.84 Patentblatt 84/14**

(45) Bekanntmachung des Hinweises auf die Patenterteilung : **22.01.86 Patentblatt 86/04**

(84) Benannte Vertragsstaaten : **BE DE FR GB IT NL**

(56) Entgegenhaltungen :
**DE-A- 1 793 426**
**DE-A- 2 061 732**
**DE-A- 2 635 327**
**FR-M- 7 701**
**US-A- 3 453 323**
**US-A- 3 802 863**
**US-A- 3 862 974**
**US-A- 4 178 169**

(73) Patentinhaber : **BASF Aktiengesellschaft**
**Carl-Bosch-Strasse 38**
**D-6700 Ludwigshafen (DE)**

(72) Erfinder : **Husslein, Gerd, Dr.**
**Rubensstrasse 36**
**D-6700 Ludwigshafen (DE)**
Erfinder : **Liesner, Michael, Dr.**
**Haardtstrasse 6**
**D-6714 Weisenheim (DE)**
Erfinder : **Hamprecht, Gerhard, Dr.**
**Rote-Turm-Strasse 28**
**D-6940 Weinheim (DE)**
Erfinder : **Varwig, Juergen, Dr.**
**Bitterstrasse 21**
**D-6900 Heidelberg (DE)**
Erfinder : **Jung, Johann, Dr. Dipl.-Landwirt**
**Hardenburgstrasse 19**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 104 473 B1

**Beschreibung**

Die Erfindung betrifft neue substituierte Phenoxyalkansäureverbindungen mit wachstumsregulierender Wirkung, Mittel, die diese Verbindungen enthalten, Verfahren zur Regulierung des Pflanzenwachstums mit diesen Verbindungen und Verfahren zur Herstellung dieser Verbindungen.

Es ist bekannt, Phenoxyalkansäuren wie beispielsweise 2,4-Dichlorphenoxyessigsäure oder 2-Methyl-4-chlor-phenoxypropionsäure als Herbizide gegen zweikeimblättrige Unkräuter (Dicotyletonen) zu verwenden, d. h. der wachstumsregulierende Effekt dieser Stoffe führt zu Eingriffen in den pflanzlichen Stoffwechsel, die letztlich das Absterben unerwünschter Dicotyledonen verursachen.

Besonders hinzuweisen ist in diesem Zusammenhang auf Pflanzenwachstumsregulatoren bzw. Herbizide, die als Sulfide, Sulfoxide oder Sulfone von Phenoxyalkansäuren zu bezeichnen sind und in der US-PS 4,178,169 beschrieben werden. Ähnliche Verbindungen, die herbizid wirksam sein sollen, sind in der US-PS 3,862,974 beschrieben. Diese Beschreibungen zeigen, daß der Übergang von stark phytotoxischer zu wachstumsregulierender Wirkung in der betrachteten Gruppe unscharf ist. Es besteht daher die Aufgabe, Sulfide, Sulfoxide oder Sulfone von Phenoxyalkansäuren zu finden, die eindeutig nur wachstumsregulierende Wirkung aufweisen, d. h. nur eine geringe Phytotoxizität besitzen.

Es wurde nun gefunden, daß Verbindungen der allgemeinen Formel I

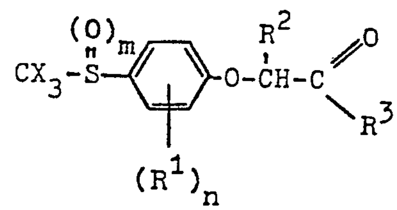

(I)

worin

$R^1$ Wasserstoff, Halogen, $C_1$-$C_4$-alkyl, $C_1$-$C_5$-alkoxy, Nitro, Cyano, Trifluormethyl

$R^2$ Wasserstoff, $C_1$-$C_3$-alkyl

$R^3$ die Gruppen OM, $OR^4$,

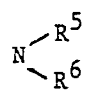

bedeutet,

worin

M für ein Äquivalent eines Metallions oder ein gegebenenfalls substituiertes Ammoniumion,

$R^4$ für einen $C_1$-$C_{12}$-Alkylrest, der gegebenenfalls durch eine oder mehrere Hydroxy-, Alkoxy-, Aminogruppen oder ein oder mehrere Halogenatome substituiert sein kann, einen gegebenenfalls in gleicher Weise substituierten Alkenyl-, Alkinyl-, Cycloalkyl-, Cycloalkenyl- oder Arylalkylrest,

$R^5$ für Wasserstoff oder für die von $R^4$ bezeichneten Reste und

$R^6$ für die von $R^5$ bezeichneten Reste und eine $C_1$-$C_6$-Alkoxygruppe und eine $C_1$-$C_6$-Alkylamino- oder Dialkylaminogruppe steht, und außerdem $R^5$ und $R^6$ zusammen mit dem benachbarten Stickstoff ein gegebenenfalls substituiertes heterocyclisches Ringsystem bilden können,

und

jedes X unabhängig voneinander Halogen oder Wasserstoff,

m gleich 0, 1 oder 2, und

n gleich 0, 1, 2, 3 oder 4 ist,

es ermöglichen, das Wachstum von Kulturpflanzen in erwünschter Weise zu verändern, ohne daß es bei den hierbei erforderlichen Aufwandmengen zu den oben erwähnten, die Pflanze schädigenden Wuchsveränderungen (beispielsweise Verdickung, Verkrümmung und teilweises Aufplatzen des Stengels, sog. « Auxinhabitus ») kommt. Die erfindungsgemäßen Verbindungen stellen somit eine wertvolle Bereicherung der Technik dar.

In der Formel I steht $R^1$ vorzugsweise für Halogen, insbesondere Fluor oder Chlor, oder für Methyl, Ethyl, n-Propyl, Isopropyl, n-Butyl, Isobutyl, tert.-Butyl, $R^2$ bedeutet vorzugsweise Wasserstoff oder Methyl, $R^3$ steht bevorzugt für OM und die diejenigen oben angegebenen Gruppen, in denen $R^4$ vorzugsweise einen $C_1$-$C_{10}$-Alkylrest, $C_1$-$C_8$-Alkenylrest, $C_1$-$C_8$-Alkinylrest, $C_3$-$C_6$-Cycloalkyl, $C_5$-$C_6$-Cycloalkenylrest oder einen Aralkylrest mit 6 bis 12 Kohlenstoffatomen im Arylteil und 1 bis 2 Kohlenstoffatomen im Alkylteil bezeichnet, die vorzugsweise durch 1 bis 3 Hydroxy- und/oder Aminogruppen, 1 bis 2 $C_1$-$C_4$-Alkoxygruppen, 1 bis 2 $C_1$-$C_4$-Monoalkylamino-, 1 bis 2 $C_2$-$C_8$-Dialkylaminoreste oder 1 bis 6 Halogenatome, insbesondere Fluor-, Chlor- oder/und Bromatome, substituiert sein können, beispielsweise Methyl, Ethyl, n-Propyl, Isopropyl, Cyclopropyl, n-Butyl, Isobutyl, tert.-Butyl, Cyclobutyl, n-Pentyl, 2-Pentyl, 3-Pentyl, tert.-Amyl, Neopentyl, 2-Methyl-butyl, 3-Methyl-butyl, 3-Methyl-2-butyl, Cyclopentyl, n-Hexyl, 4-Methyl-2-pentyl, 2,3-Dimethylbutyl, 2-Methyl-1-pentyl, 2-Hexyl, 3-Hexyl, 3-Methyl-2-pentyl, 3-Methylpentyl, 4-Methylpentyl, 3-Methyl-3-pentyl, 4,4-Dimethylbutyl, Cyclohexyl, Heptyl, 2-Heptyl, 3-Heptyl, 4-Heptyl, Cycloheptyl, 1-Octyl, 2-Octyl, 3-Octyl, 4-Octyl, 5-Octyl, 5-Ethyl-2-heptyl, 2,6-Dimethyl-4-heptyl, 7-Ethyl-2-methyl-4-nonyl, 2,4-Dimethyl-3-pentyl, 3-Methyl-2-heptyl, 5-Ethyl-2-nonyl, Nonyl, Decyl, 6-Ethyl-3-octyl, 2-Methyl-2-pentyl, 2,3-Dimethyl-2-butyl, 2-Methyl-2-hexyl, 3-Ethyl-3-pentyl, 3-Methyl-3-hexyl, 2,3-Dimethyl-pentyl-3,2,4-Dimethyl-2-pentyl, 2,2,3-Trimethyl-3-butyl, 2-Methyl-2-heptyl, 4-Methyl-4-heptyl, 2,4-Dimethyl-2-hexyl, 2-Methyl-2-octyl, 1-Methyl-1-cyclopentyl, 1-Methyl-1-cyclohexyl, 1-Ethyl-1-cyclohexyl, Chlor-tert.-butyl, 1,1-Dichlor-2-methyl-2-propyl, 1,3-Dichlor-2-methyl-2-propyl, 1-Cyclohexyl-1-ethyl, 1-Chlorethyl, 2-Chlorethyl, 1-Chlorpropyl, 2-Chlorpropyl, 3-Chlorpropyl, 1-Chlor-2-propyl, 2-Chlorbutyl, 2-Chlor-2-methyl-3-propyl, 1-Fluorethyl, 2-Fluorethyl, 2-Fluorpropyl, 3-Fluorpropyl, 1-Fluor-2-propyl, 2-Fluorbutyl, 2-Fluor-2-methyl-3-propyl, 2-Bromethyl, 3-Brompropyl, 4-Chlorbutyl, 2-Chlorcyclohexyl, 1,1,1-Trifluorisopropyl, Hexafluor-2-methyl-isopropyl, Hexafluorisopropyl, Hexachlorisopropyl, 1,2-Dibromallyl, 2,2,2-Trifluorethyl, 1-Chlorbutin-2-yl-4, 3-Chlor-butin-1-yl-4, 1-Chlor-buten-2-yl-4, 2,3-Dibrom-1-propyl, 2,2,2-Trichlorethyl, 1-Chlorpentin-2-yl-4, 2,2,2-Tribromethyl, 3,4,4-Trichlorbuten-3-yl-2, 1-Brom-2-propyl, 1,3-Dibrom-2-propyl, 3-Chlorbuten-1-yl-4, Allyl, Methallyl, Crotyl, 2-Ethyl-hexen-2-yl-1, Hexen-5-yl-1, 2-Methyl-buten-2-yl-1, 2-Methylbuten-1-yl-3, Butin-1-yl-3, Butin-2-yl-1, Buten-1-yl-3, Propargyl, 2-Methylbuten-1-yl-4, 2-Methyl-buten-2-yl-4, 3-Methyl-buten-1-yl-3, 1-Ethinylcyclohexyl, Methoxyethyl, Ethoxyethyl, 3-Methoxypropyl, Methoxyisopropyl, 3-Methoxybutyl, 1-Methoxybutyl-2, Ethoxy-tert.-butyl, Methoxy-tert.-butyl, Cyclohexoxy-tert.-butyl, 2-Methoxy-butyl, 4-Methoxy-butyl, 2,2-Dimethoxyethyl, 2-Dimethylaminoethyl, 2-Dipropylaminoethyl, 2-Diisopropylaminoethyl, 2-(2'-Ethoxyethyl)-aminoethyl, Cyclohexenyl-1, Cyclopentyl-3,2-Hydroxyethyl, 2-Hydroxypropyl, 2,3-Dihydroxypropyl, 2-(2'-Hydroxy)-ethoxyethyl, α-Phenylethyl, α-o,p-Dichlorphenylethyl, β-Phenylethyl, β-p-Bromphenylethyl, α-Naphthylmethyl, 2-Methylaminoethyl, 2-Ethylaminoethyl, 3-Isopropylaminopropyl, 2-Aminoalkyl, 1-Methyl-2-aminoethyl, 2'-Aminoethoxyethyl, 2'-Aminoethylaminoethyl.

Als Beispiele für M seien angeführt ein Alkalimetallion, beispielsweise Natrium, Kalium, ein Äquivalent eines Erdalkaliions, beispielsweise Magnesium, Calcium, ein gegebenenfalls substituiertes Ammoniumion, beispielsweise ein Methylammonium-, Dimethylammonium-, 2-Hydroxyethylammonium-, 2-Trihydroxyethylammoniumion.

$R^5$ steht bevorzugt für Wasserstoff und die oben für $R^4$ als bevorzugt angegebenen Gruppen.

$R^6$ steht bevorzugt für Wasserstoff, die für $R^4$ oben als bevorzugt angegebenen Reste sowie eine $C_1$-$C_3$-Alkoxygruppe, eine $C_1$-$C_2$-Monoalkylamino- oder $C_2$-$C_4$-Dialkylaminogruppe. Bilden $R^5$ und $R^6$ zusammen mit dem benachbarten Stickstoff ein gegebenenfalls substituiertes Ringsystem, bedeuten sie vorzugsweise einen 5- bis 6-gliedrigen Heterocyclus, der gegebenenfalls Doppelbindungen, weitere Heteroatome, insbesondere Stickstoff, Sauerstoff und/oder Schwefel enthalten sowie einen oder mehrere Substituenten, insbesondere Halogenatome und/oder Methylgruppen tragen kann. Als Beispiele für $R^5$ bzw. $R^6$ seien genannt: Wasserstoff, die unter $R^4$ beispielhaft angeführten Reste, Methoxy, Ethoxy, Propoxy, Isopropoxy, Methylamino, Dimethylamino, Diethylamino, ein Pyrrolidinyl-, 4-Methylpiperazinyl-, Imidazol-, 3,5-Dimethylpyrazolyl-, 2,6-Dimethylmorpholinylrest.

Man kann die erfindungsgemäßen Verbindungen der Formel I erhalten, indem man entweder

a) Verbindungen der allgemeinen Formel II

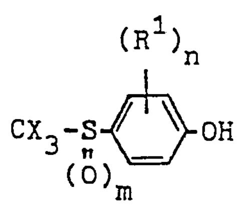

(II)

worin $R^1$, X, m und n die oben angegebenen Bedeutungen besitzen, oder deren Salze, mit einer Verbindung der Formel III

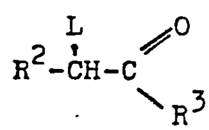

(III)

worin $R^2$ und $R^3$ die oben angegebenen Bedeutungen haben und L einen nucleophil verdrängbaren Substituenten bedeutet,
gegebenenfalls in Gegenwart eines Verdünnungsmittels und/oder einer anorganischen oder organischen Base bei Temperaturen zwischen 10 °C und dem Siedepunkt des Gemisches gegebenenfalls in Gegenwart eines Reaktionsbeschleunigers umsetzt oder ein

b) Methylthiophenol der Formel IV

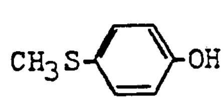

(IV)

mit einer Verbindung der Formel III zu einer Verbindung der Formel V

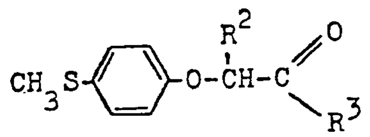

(V)

gegebenenfalls in Gegenwart eines Verdünnungsmittels und/oder einer anorganischen oder organischen Base bei Temperaturen zwischen 20 °C und dem Siedepunkt des Gemisches gegebenenfalls in Gegenwart eines Reaktionsbeschleunigers umsetzt und diese mit einem Halogenierungsmittel in Verbindungen der Formel VI

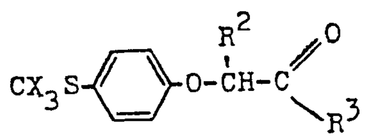

(VI)

worin X die oben genannten Bedeutung hat, gegebenenfalls in Gegenwart eines Verdünnungsmittels bei Temperaturen zwischen 0 °C und dem Siedepunkt des Gemisches gegebenenfalls in Gegenwart eines Reaktionsbeschleunigers überführt.

Verwendet man p-Trifluormethylsulfonyl-phenol und α-Brompropionsäuremethylester als Ausgangsstoffe, kann der Reaktionsablauf des erfindungsgemäßen Verfahrens a) durch folgendes Formelschema wiedergegeben werden :

a) 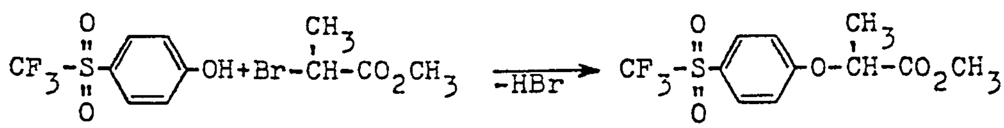


Verwendet man p-Methylmercapto-phenol und Chloressigsäuremethylester als Ausgangsmaterialien und Chlor als Halogenierungsmittel, kann der Ablauf des erfindungsgemäßen Verfahrens b) durch folgendes Reaktionsschema wiedergegeben werden :

b1) 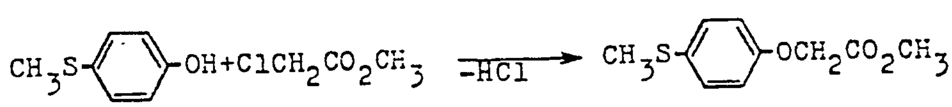


b2) 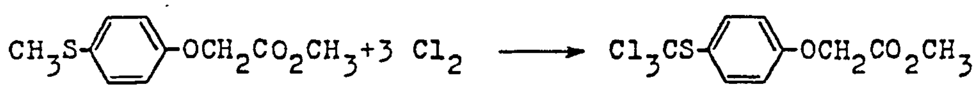

Eine so erhaltene Verbindung der allgemeinen Formel VI kann gegebenenfalls am Schwefelatom oxidiert werden, beispielsweise

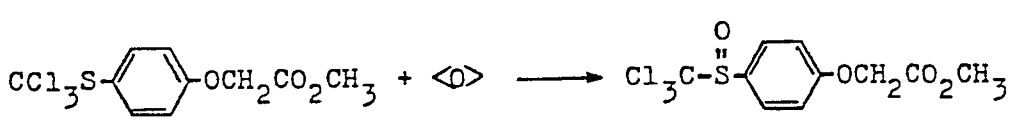

und/oder mit Resten $R^1$ versehen werden, beispielsweise

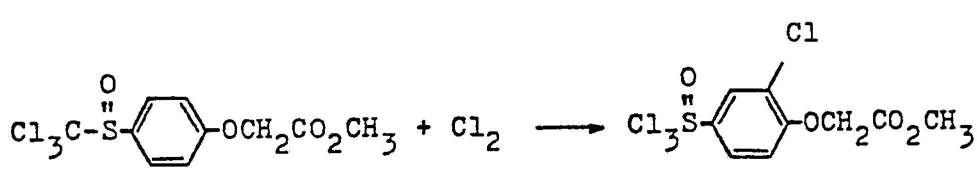

Die zur Herstellung der Verbindungen der Formel I mit n, m O notwendige Einführung der Substituenten $R^1$ bzw. Oxidation des Schwefelatoms kann je nach der chemischen Verträglichkeit dieser Maßnahmen mit den am Molekül bereits vorhandenen Gruppen an jeder dem Fachmann zweckmäßig erscheinenden Stelle des Syntheseschemas vorgenommen werden.

Für den im Verfahren a) erwähnten nucleophil verdrängbaren Substituenten seien beispielsweise genannt: Halogen, wie Chlor, Brom oder Jod, Hydrogensulfat, Hydrogensulfonat, gegebenenfalls substituierte Alkylsulfonyloxy-, Arylsulfonyloxy-, Alkylsulfat-, Phenoxy-, Phenylthio-, Oxomium-, Sulfonium- oder Ammonium-Reste.

Geeignete anorganische oder organische Basen, die gegebenenfalls auch als Säure-bindende Mittel beim Verfahren a) und b) eingesetzt werden können, sind beispielsweise Kaliumhydroxid, Natriumhydroxid, Kaliumcarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Zinkhydroxid, Zinkoxid, Zinkcarbonat, Zinkbicarbonat, Natriummethylat, Magnesiummethylat, Kaliumethylat, Natriumpropylat, Aluminiumisopropylat, Natriumbutylat, Lithiummethylat, Kaliumcyclohexanolat, Natriumisopropylat, Kalium-tert.-butylat, Trimethylamin, Triethylamin, Tripropylamin, Triisopropylamin, Tributylamin, Triisobutylamin, Tri-sek-butylamin, Tri-tert.-butylamin, Tribenzylamin, Tricyclohexylamin, Triamylamin, Trihexylamin, N,N-Dimethylanilin, N,N-Diethylanilin, N,N-Diisopropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N-Methylpiperidin, N-Ethylpiperidin, N-Methylpyrrolidin, N-Ethylpyrrolidin, N-Methylpyrrol, N-Methylmorpholin, N-Ethylmorpholin, N-Methylhexamethylenimin, Pyridin, Chinolin, α,β,γ-Picolin, N,N,N',N'-Tetramethylethylendiamin, N-Ethyldiisopropylamin, N,N-Dimethylcyclohexylamin, Natriumhydrid, Lithiumhydrid, Calciumhydrid. Es können aber auch andere, üblicherweise verwendete basische Stoffe verwendet werden.

Soll das Verfahren a) und der erste Schritt des Verfahrens b) in Gegenwart eines Verdünnungsmittels vorgenommen werden, gehören zu den bevorzugten Verdünnungsmitteln polare Lösungsmittel wie Wasser; Formamide, wie Dimethylformamid, Formamid, Dimethylacetamid; Nitrile, wie Acetonitril, Benzonitril, Butyronitril; Sulfoxide, wie Dimethylsulfoxid; Phosphorsäureamide, wie Hexamethylphosphorsäuretriamid; Ketone, wie Aceton, Ethylmethylketon, Cyclohexanon, Acetophenon; Ether, wie Tetrahydrofuran, Anisol, Dimethoxyethan, n-Butylethylether, Dioxan; Nitroalkane, wie Nitromethan; Nitrobenzol; Alkohole, wie Methanol, Ethanol, Isopropanol, n-Butanol, 3-Methylbutanol; Harnstoffe wie Tetramethylharnstoff; Etheralkohole, wie Ethylenglykolmonomethylether; Sulfone, wie Sulfolan; Ester, wie Essigsäureethylester, Propionsäuremethylester, Ameisensäuremethylester. Daneben kommen jedoch auch in Frage: Halogenkohlenwasserstoffe, insbesondere Chlorkohlenwasserstoffe, beispielsweise Methylenchlorid, Chloroform, 1,2-Dichlorethan, 1,1,2,2- oder 1,1,1,2-Tetrachlorethan, Dichlorpropan, Trichlorethylen, Chlorbenzol, o,m,p-Dichlorbenzol, Fluorbenzol, o,m,p-Chlortoluol, Dichlornaphthalin, Tetrachlorkohlenstoff; aliphatische oder cycloaliphatische Kohlenwasserstoffe, wie Heptan, Pinan, o,m,p-Cymol, Benzinfraktionen innerhalb eines Siedepunktintervalles von 70 bis 190 °C, Cyclohexan, Methylcyclohexan, Dekalin, Petrolether, Ligroin, 2,2,4-Trimethylpenten, Octan; aromatische Kohlenwasserstoffe, wie Benzol, Toluol, o,m,p-Xylol, Tetralin, und entsprechende Gemische. Zweckmäßig verwendet man das Lösungsmittel in einer Menge von 100 bis 2 000 Gew.%, vorzugsweise 100 bis 1 000 Gew.%, bezogen auf die Ausgangsstoffe (II) bzw. (IV).

Soll der Halogenierungsschritt des Verfahrens b) in Gegenwart eines Verdünnungsmittels vorgenommen werden, kommen hierzu bevorzugt unpolare Verdünnungsmittel in Frage: Halogenkohlenwasserstoffe, insbesondere Chlorkohlenwasserstoffe, beispielsweise Methylenchlorid, Chloroform, 1,2-Dichlorethan, 1,1,2,2- oder 1,1,1,2-Tetrachlorethan, Dichlorpropan, Trichlorethylen, Tetrachlorkohlenstoff; aliphatische oder cycloaliphatische Kohlenwasserstoffe wie Heptan, Hexan, Pentan, Pinan, o,m,p-Cymol, Benzinfraktionen innerhalb eines Siedeintervalles von 70 bis 190 °C, Cyclohexan, Methylcyclohexan, Decalin, Petrolether, Ligroin, 2,2,4-Trimethylpentan, Octan. Daneben können aber auch polare Lösungsmittel verwendet werden, wie Wasser; Formamide, wie Dimethylformamid, Formamid, Dimethylacetamid; Nitrile, wie Acetonitril, Benzonitril, Butyronitril; Phosphorsäureamide, wie Hexamethylphosphorsäuretriamid; Ketone, wie Aceton, Ethylmethylketon, Cyclohexanon, Acetophenon; Ether, wie Tetrahydrofuran, Anisol, Dimethoxyethan, n-Butylethylether, Dioxan; Nitroalkane, wie Nitromethan; Nitrobenzol; Alkohole, wie Methanol, Ethanol, Isopropanol, n-Butanol, 3-Methylbutanol; Etheralkohole, wie Ethylenglykolmonomethylether; Ester, wie Essigsäuremethylester, Propionsäuremethylester, Ameisensäuremethylester.

Im erfindungsgemäßen Verfahren a) und dem ersten Teilschritt des Verfahrens b) kommen als Reaktionsbeschleuniger vorzugsweise Metall-Jodide und -bromide, beispielsweise Natriumjodid, Kaliumbromid, Calciumjodid; Kronenether; quartäre Ammoniumverbindungen, wie Tetrabutylammoniumjodid oder -bromid; Säuren oder Kombinationen dieser Stoffe in Frage.

Im zweiten Schritt des Verfahrens b) kommen bevorzugt Alkalicarbonate und Alkalialkoholate, beispielsweise Natriumcarbonat, Kaliumcarbonat, Natriummethylat, Natriumethylat, daneben aber auch aliphatische, aromatische oder heterocyclische Amine, beispielsweise Triethylamin, Dimethylamin, Dimethylanilin, Pyridin, sowie Licht, beispielsweise sichtbares Licht, und eine Kombination dieser Maßnahmen als Reaktionsbeschleuniger in Frage.

Als Halogenierungsmittel kommen im zweiten Schritt des Verfahrens b) bevorzugte Halogene, insbesondere Chlor, Brom; anorganische Säurehalogenide, insbesondere Sulfurylchlorid, Phosphortribromid, Phosphoroxytrichlorid, Phosphorpentachlorid, Thionylchlorid; Halogenamide oder -imide, beispielsweise N-Chlorsuccinimid, N-Bromsuccinimid, N-Chloracetamid, Chloramin T sowie Kombinationen dieser Stoffe in Frage. Zur Herstellung der Verbindungen I mit X = F schließt sich im allgemeinen

einer derart durchgeführten Halogenierung in grundsätzlich bekannter Weise eine Umsetzung mit Fluorwasserstoff an.

Soll eine Verbindung der allgemeinen Formel VI am Schwefelatom oxidiert werden, kommen hierzu vorzugsweise die üblicherweise verwendeten Peroxiverbindungen, beispielsweise Wasserstoffperoxid, Peressigsäure, m-Chlorperbenzoesäure in Frage, gegebenenfalls in Gegenwart eines Verdünnungsmittels, vorteilhaft Wasser, Carbonsäuren wie Ameisensäure, Essigsäure, Propionsäure, Ketone, wie Aceton, Methylethylketon, Cyclohexanon, Halogenkohlenwasserstoffe, wie Methylenchlorid, 1,2-Dichlorethan, Chloroform, aliphatische und cycloaliphatische Kohlenwasserstoffe wie Hexan, Heptan, Pinan, Cyclohexan, Ligroin, Petrolether, Ester wie Ameisensäureethylester, Essigsäureethylester, gegebenenfalls in Gegenwart eines Reaktionsbeschleunigers, beispielsweise, Schwefelsäure, wobei die Reaktionstemperatur üblicherweise zwischen 0 °C und dem Siedepunkt des Gemisches liegt.

Für die Einführung von Substituenten $R^1$ an geeigneten Stellen des Verfahrensablaufs gelten die wohlbekannten Regeln bzw. experimentellen Verfahren der elektrophilen aromatischen Substituenten, vgl. beispielsweise Houben-Weyl, Band V/3 (1962), 651-725, ibid. V/4 (1960), 233-331 und 557-594.

In einer bevorzugten Ausführungsform vermischt man in beliebiger Reihenfolge ein Phenol der allgemeinen Formel II und eine Verbindung der Formel III, gegebenenfalls ein Verdünnungsmittel und eine Base und einen Reaktionsbeschleuniger und setzt das Gemisch bei einer Temperatur zwischen 20 °C und dem Siedepunkt für eine Zeit von i. a. weniger als 30 Stunden, drucklos oder unter Druck, kontinuierlich oder diskontinuierlich um.

Im allgemeinen verwendet man auf 1 mol der Stoffe II bzw. IV 0,9 bis 2,5 mol des Stoffes III, sowie 0,8 bis 5 mol Base, verwendet man einen Reaktionsbeschleuniger, werden davon auf 1 mol II bzw. IV 0,001 bis 0,1 mol eingesetzt. Verwendet man ein Verdünnungsmittel, setzt man davon i. a. weniger als 1 000 Gew.%, bezogen auf den Ausgangsstoff II bzw. IV, ein. Besonders bevorzugt ist folgende Verfahrensweise : Man legt die Ausgangsstoffe II bzw. IV in einem Verdünnungsmittel wie Aceton, Methylethylketon vor, setzt dann eine Base wie Natriumcarbonat, Kaliumcarbonat zu und gibt dann die Verbindung III zu und läßt das Gemisch bei 40 °C bis dem Siedepunkt während 0,5 bis 10 Stunden drucklos abreagieren. Zur Isolierung der Stoffe I bzw. V dekantiert man von der Base, trocknet das produkthaltige Verdünnungsmittel, filtriert und zieht das Verdünnungsmittel ab. Das zurückbleibende Produkt kann gegebenenfalls nach bekannten Methoden wie Umkristallisation, Destillation oder Chromatographie weiter gereinigt werden.

Zur Halogenierung einer so dargestellten Verbindung V vermischt man in einer bevorzugten Ausführungsform den Ausgangsstoff mit einem Verdünnungsmittel und versetzt das Gemisch bei Temperaturen von 0 °C bis zum Siedepunkt mit einer bis zur Erreichung des gewünschten Halogenierungsgrades erforderlichen Menge des Halogenierungsmittels. Die Reaktionsdauer richtet sich nach dem Verbrauch des Halogenierungsmittels durch den Ausgangsstoff V und liegt zwischen 30 Minuten und 20 Stunden. In einer besonders bevorzugten Ausführungsform der erfindungsgemäßen Halogenierung vermischt man den Stoff V mit einem inerten Halogen-Kohlenwasserstoff und gibt bei Temperaturen zwischen 10 °C und dem Siedepunkt des Verdünnungsmittels die stöchiometrisch erforderliche Menge Chlor oder Brom zu. Nach der Entfärbung der Lösung isoliert man den Endstoff durch Abziehen des Verdünnungsmittels und gegebenenfalls durch weitere Reinigung durch Kristallisation, Destillation oder Chromatographie. Zur Herstellung einer Fluorverbindung VI (X = F) kann man die organische Lösung bei − 10 °C bis zum Siedepunkt des Verdünnungsmittels mit Fluorwasserstoff vermischen. Fluorwasserstoff wird im allgemeinen in einer Menge von 0,8 bis 20 Mol pro Mol Ausgangsstoff, verdünnt oder unverdünnt, mit oder ohne Reaktionsbeschleuniger wie Bortrifluorid, Aktivkohle, Aluminiumchlorid, Titantetrachlorid mit oder ohne Druck angewandt, jedoch kann auch eine erheblich größere Menge eingesetzt werden, beispielsweise wenn Fluorwasserstoff als Lösungsmittel verwendet wird. Nach der Entfernung des Verdünnungsmittels und/oder überschüssigen Fluorwasserstoffs kann der Endstoff nach bekannten Methoden, beispielsweise Kristallisation, Destillation oder Chromatographie weiter gereinigt werden.

Die Ausgangsstoffe I sind z. T. bekannt bzw. können nach bekannten Methoden dargestellt werden ; vgl. beispielsweise DE-PS 1 257 784 ; Fr-PS 1 572 160 ; FR-PS 2 280 364 ; DE-PS 2 117 574 ; Yagupolsky L.M. und Maranets M.S., Zh. Obshch. him. 24 (1954), 887.

Die Ausgangsstoffe der Formel IV sind ebenfalls z. T. bekannt bzw. können nach bekannten Methoden hergestellt werden, vgl. beispielsweise DE-PS 2 644 591 ; JA-PS 9134 ('67) (CA 68 (1968), 68709x) ; JA-PS 6723, 415 (CA 69 (1968) 35738y).

Herstellbeispiel 1

Zu einer Mischung von 70 g 2-Chlor-4-(trifluormethylthio)-phenol und 100 g wasserfreiem Kaliumcarbonat in 250 g Methylethylketon wurden unter kräftigem Rühren 60 g 2-Brompropionsäuremethylester gegeben und 5 Stunden am Rückfluß gehalten. Danach wurde dekantiert, der Rückstand zweimal mit Methylenchlorid gewaschen, die vereinigten organischen Phasen mit Wasser behandelt, und die Phasen getrennt. Man extrahierte die wäßrige Phase nochmals mit Methylenchlorid, trocknete die vereinigten Extrakte mit Magnesiumsulfat und engte ein. Nach der Destillation erhielt man 88,4 g (87 %, bezogen auf das Phenol) 2-(2'-Chlor-4'-trifluormethylthio)-phenoxypropionsäuremethylester. Kochpunkt 98 bis 101 °C/0,2 mbar.

Herstellbeispiel 2

Zu einer Mischung von 300 g p-Methylmercaptophenol und 590 g wasserfreiem Kaliumcarbonat in 2 000 g Methylethylketon wurden 314 g 2-Chlorpropionsäuremethylester und 3 g Natriumjodid gegeben. Nach 6stündigem Rühren bei Rückfluß wurde die organische Phase dekantiert, der Rückstand noch einmal mit Methylethylketon nachgewaschen und die vereinigten organischen Phasen eingeengt. Den Rückstand verteilte man zwischen Methylenchlorid und Wasser, trocknete den organischen Extrakt und zog das Lösungsmittel ab. Nach der Destillation wurden 430 g (89 % bezogen auf das Phenol) 2-[4'-Methylmercaptophenoxy]-propionsäuremethylester (Kochpunkt 125 bis 128 °C/0,5 mbar) erhalten.

Herstellbeispiel 3

In eine Lösung von 188,8 g des so erhaltenen 2-[4'-Methylmercapto]-phenoxypropionsäuremethylester in 500 g Tetrachlormethan wurden bei 25 °C innerhalb von 50 Minuten 179 g Chlorgas eingeleitet, wobei sich die Lösung auf 35 °C erwärmte. Nach weiteren 15 Minuten (Ende der HCl-Entwicklung) wurde das Lösungsmittel abgezogen. Man erhielt so 276 g (100 % der erwarteten Menge) 2-[4'-Trichlormethylthiophenoxy]-propionsäuremethylester (Schmelzpunkt 73 bis 75 °C ; Kochpunkt 154 bis 156 °C/0,5 mbar).

Herstellbeispiel 4

Zu einer Lösung von 20 g 2-[4'-Trichlormethylthio]-phenoxypropionsäuremethylester in 20 g Eisessig wurden 0,3 g Diphenylsulfid und dann ohne äußere Kühlung 9 g Sulfurylchlorid zugesetzt. Man rührte 4 Stunden nach, zog das Lösungsmittel ab und destillierte den Rückstand im Vakuum. Man erhielt so 13,7 g (62 % der erwarteten Menge) 2-[2'-Chlor-4'-trichlormethylthiophenoxy]-propionsäuremethylester. Kochpunkt 170 bis 172 °C/0,4 mbar.

Herstellbeispiel 5

Zu 160 g 2-[4'-Trichlormethylthio]-phenoxypropionsäuremethylester wurden bei − 80 °C 400 g wasserfreier Fluorwasserstoff zugesetzt. Nach 5stündigem Rühren unter Rückfluß wurde überschüssiger Fluorwasserstoff abgezogen, der Rückstand in 300 g methylenchlorid aufgenommen, mit 10 g Kaliumfluorid versetzt, filtriert, eingeengt und im Vakuum destilliert. Man erhielt so 116 g (85 % der Theorie) 2-[4'-Trifluormethylthiophenoxy]-propionsäuremethylester. Kochpunkt 85 bis 88 °C/0,1 mbar.

Herstellbeispiel 6

In eine Lösung von 200 g 2-[2'-Chlor-4'-trifluormethylthiophenoxy]-propionsäuremethylester in 2 000 g Methylenchlorid wurden bei 0 bis 10 °C 138 g m-Chlorperbenzoesäure (80 %ig) eingetragen und dann 48 Stunden bei Raumtemperatur nachgerührt. Danach wurde abgesaugt, der Niederschlag gründlich mit Methylenchlorid nachgewaschen, Filtrat und Waschflüssigkeit zusammen mit gesättigter wäßriger Natriumhydrogencarbonat-Lösung extrahiert, die organische Phase getrocknet und das Lösungsmittel abgezogen. Man erhielt so 187 g (87 % der Theorie) 2-[2'-Chlor-4'-trifluormethylsulfinylphenoxy]-propionsäuremethylester, $n_D^{20}$ = 1,506 0.

Herstellbeispiel 7

Verfährt man wie vorstehend beschrieben und verwendet 275 g m-Chlorperbenzoesäure auf 200 g 2-[2'-Chlor-4'-trifluormethylthio]-phenoxypropionsäuremethylester, so erhält man 209 g (93 % der Theorie) 2-[2'-Chlor-4'-trifluormethylsulfonylphenoxy]-propionsäuremethylester vom Schmelzpunkt 48 bis 52 °C.

Durch entsprechende Abwandlung der vorstehenden praktischen Angaben kann man die in der nachstehenden Tabelle aufgeführten Verbindungen erhalten ; soweit sie hergestellt und näher untersucht wurden, sind ihre physikalischen Eigenschaften (Schmelzpunkt, Siedepunkt, Brechungsindex, Drehwert, wenn optisch aktiv) angegeben. Die nicht näher charakterisierten Verbindungen können durch entsprechende Abwandlung und Wahl anderer Ausgangsstoffe erhalten werden ; aufgrund struktureller Überlegungen lassen sie eine ähnliche biologische Wirkung erwarten, wie die untersuchten Verbindungen.

(Siehe Tabelle Seite 8 ff.)

Tabelle

| Beispiel Nr. | X | m | $(R^1)_n$ | $R^2$ | $R^3$ | Schmp. °C; Sdp. °C/mbar | $[\alpha]_D^{20}$ $n_D^{20}$; Wellenlänge einer Bande im IR-Spektrum |
|---|---|---|---|---|---|---|---|
| 8 | F | 2 | H | $CH_3$ | $OCH_3$ | 115/0,1 | |
| 9 | Cl | 1 | H | $CH_3$ | $OCH_3$ | 98-102 | |
| 10 | H | 1 | H | $CH_3$ | $OCH_3$ | | 1.5432 |
| 11 | F | 1 | H | $CH_3$ | $OCH_3$ | | 1.4967 |
| 12 | H | 0 | H | H | $OCH_3$ | Öl | 1496 $cm^{-1}$ |
| 13 | H | 2 | H | $CH_3$ | $OCH_3$ | 114-115 | |
| 14 | H | 2 | H | $CH_3$ | $OCH_3$ | 71- 73 | |
| 15 | H | 0 | H | $C_2H_5$ | $OCH_3$ | | |
| 16 | H | 0 | H | $C_3H_7$ | $OCH_3$ | | |
| 17 | H | 0 | H | $CH(CH_3)_2$ | $OCH_3$ | | |
| 18 | H | 0 | H | $CH_3$ | $OC_4H_9n$ | | |
| 19 | H | 1 | H | H | $OCH_3$ | 46- 47 | |
| 20 | $F_2H$ | 0 | H | $CH_3$ | $OCH_3$ | 98-101/0,2 | |
| 21 | F | | H | $CH_3$ | OH | 93- 95 | |
| 22 | H | 0 | 2-Cl | $CH_3$ | $OCH_3$ | | 1.5555 |
| 23 | H | 0 | 2-Cl | $CH_3$ | $OCH_3$ | | $[\alpha]_D^{20} = + 42.6$ |
| 24 | H | 0 | 2-Cl | H | $OCH_3$ | | |
| 25 | H | 0 | 2-Cl | $C_2H_5$ | $OCH_3$ | | 1.5490 |
| 26 | H | 0 | 2-Cl | $C_3H_7$ | $OCH_3$ | | |
| 27 | H | 0 | 2-Cl | $CH(CH_3)_2$ | $OCH_3$ | | |

Tabelle : Fortsetzung

| Beispiel Nr. | X | m | $(R^1)_n$ | $R^2$ | $R^3$ | Schmp. °C; Sdp. °C/mbar | $[\alpha]_D^{20}$ $n_D^{20}$; Wellenlänge einer Bande im IR-Spektrum |
|---|---|---|---|---|---|---|---|
| 28 | H | 0 | 2-Cl | $CH_3$ | OH | 93- 96 | |
| 29 | H | 0 | 2-Cl | $CH_3$ | OK | 102-106 | |
| 30 | H | 1 | 2-Cl | $CH_3$ | $OCH_3$ | | 1.5514 |
| 31 | Cl | 1 | 2-Cl | $CH_3$ | $OCH_3$ | | 1.5775 |
| 32 | H | 2 | 2-Cl | $CH_3$ | $OCH_3$ | 94- 97 | |
| 33 | Cl | 2 | 2-Cl | $CH_3$ | $OCH_3$ | 80- 90 | |
| 34 | H | 0 | 2-Cl | $CH_3$ | $N(CH_3)_2$ | | |
| 35 | H | 0 | 2-Cl | $CH_3$ | $NHCH(CH_3)_2$ | | |
| 36 | H | 0 | 2-Cl | $CH_3$ | $NH_2$ | | |
| 37 | H | 0 | 2-$CH_3$ | $CH_3$ | $OCH_3$ | | 1.5440 |
| 38 | H | 2 | 2-$CH_3$ | $CH_3$ | $OCH_3$ | 58- 61 | |
| 39 | H | 2 | 2-$CH_3$ | $CH_3$ | OH | | |
| 40 | H | 0 | 2-$CH_3$ | H | OH | | |
| 41 | H | 0 | 2-$CH_3$ | $C_2H_5$ | $OCH_3$ | | |
| 42 | H | 0 | 2-$CH_3$ | $C_2H_5$ | OH | | |
| 43 | Cl | 0 | 2-$CH_3$ | $CH_3$ | $OCH_3$ | | 1.5617 |
| 44 | Cl | 2 | 2-$CH_3$ | $CH_3$ | $OCH_3$ | 83- 86 | |
| 45 | H | 0 | 2-$NO_2$ | $CH_3$ | $OCH_3$ | | |
| 46 | H | 2 | 2-$NO_2$ | $CH_3$ | $OCH_3$ | | |

Tabelle : Fortsetzung

| Beispiel Nr. | X | m | $(R^1)_n$ | $R^2$ | $R^3$ | Schmp. °C; Sdp. °C/mbar | $[\alpha]_D^{20}$ $n_D^{20}$; Wellenlänge einer Bande im IR-Spektrum |
|---|---|---|---|---|---|---|---|
| 47 | Cl | 0 | $2-NO_2$ | $CH_3$ | $OCH_3$ | | |
| 48 | Cl | 2 | $2-NO_2$ | $CH_3$ | $OCH_3$ | | |
| 49 | H | 0 | $3-CH_3$ | H | $OCH_3$ | 120/0,1 | |
| 50 | H | 0 | $3-CH_3$ | $CH_3$ | $OCH_3$ | | |
| 51 | H | 0 | $3-CH_3$ | $C_2H_5$ | $OCH_3$ | | |
| 52 | H | 1 | $3-CH_3$ | H | $OCH_3$ | 78- 79 | |
| 53 | H | 2 | $3-CH_3$ | H | $OCH_3$ | 125-126 | |
| 54 | Cl | 0 | $3-CH_3$ | $CH_3$ | $OCH_3$ | | |
| 55 | H | 1 | $3-CH_3$ | $CH_3$ | $OCH_3$ | Öl | 1481 $cm^{-1}$ |
| 56 | H | 2 | $3-CH_3$ | $CH_3$ | $OCH_3$ | Öl | 1307 $cm^{-1}$ |
| 57 | Cl | 2 | $3-CH_3$ | $CH_3$ | $OCH_3$ | | |
| 58 | H | 0 | $2,6-Cl_2$ | $CH_3$ | $OCH_3$ | | 1.5661 |
| 59 | H | 0 | $2,6-Cl_2$ | $CH_3$ | OH | 112-115 | |
| 60 | H | 0 | $2,6-Cl_2$ | $CH_3$ | OK | 84- 87 | |
| 61 | H | 1 | $2,6-Cl_2$ | $CH_3$ | $OCH_3$ | Wachs | 1445 $cm^{-1}$ |
| 62 | H | 1 | $2,6-Cl_2$ | $CH_3$ | OH | Wachs | |
| 63 | H | 2 | $2,6-Cl_2$ | $CH_3$ | $OCH_3$ | Wachs | 1448 $cm^{-1}$ |
| 64 | H | 2 | $2,6-Cl_2$ | $CH_3$ | OH | 107-110 | |
| 65 | Cl | 0 | $2,6-Cl_2$ | $CH_3$ | $OCH_3$ | | 1.5710 |
| 66 | Cl | 1 | $2,6-Cl_2$ | $CH_3$ | $OCH_3$ | Wachs | |

Tabelle : Fortsetzung

| Beispiel Nr. | X | m | $(R^1)_n$ | $R^2$ | $R^3$ | Schmp. °C; Sdp. °C/mbar | $[\alpha]_D^{20}$ $n_D^{20}$; Wellenlänge einer Bande im IR-Spektrum |
|---|---|---|---|---|---|---|---|
| 67 | Cl | 2 | 2,6-$Cl_2$ | $CH_3$ | $OCH_3$ | Wachs | |
| 68 | $F_2Cl$ | 0 | 2,6-$Cl_2$ | $CH_3$ | $OCH_3$ | | 1.5325 |
| 69 | H | 0 | 2,3-$(CH_3)_2$ | $CH_3$ | $OCH_3$ | | |
| 70 | H | 0 | 2,5-$(CH_3)_2$ | $CH_3$ | $OCH_3$ | | |
| 71 | H | 0 | 3,5-$(CH_3)_2$ | $CH_3$ | $OCH_3$ | | |
| 72 | H | 0 | 2,6-$(CH_3)_2$ | $CH_3$ | $OCH_3$ | | 1.5425 |
| 73 | H | 0 | 2,6-$(CH_3)_2$ | H | $OCH_3$ | | |
| 74 | H | 0 | 2,6-$(CH_3)_2$ | $C_2H_5$ | $OCH_3$ | | |
| 75 | Cl | 0 | 2,6-$(CH_3)_2$ | $CH_3$ | $OCH_3$ | | 1.5557 |
| 76 | Cl | 1 | 2,6-$(CH_3)_2$ | $CH_3$ | $OCH_3$ | | |
| 77 | Cl | 2 | 2,6-$(CH_3)_2$ | $CH_3$ | $OCH_3$ | 72- 75 | |
| 78 | H | 2 | 2,6-$(CH_3)_2$ | $CH_3$ | $OCH_3$ | | 1.5315 |
| 79 | H | 0 | 2-Cl-5-$CH_3$ | $CH_3$ | $OCH_3$ | | |
| 80 | H | 2 | 2-Cl-5-$CH_3$ | $CH_3$ | $OCH_3$ | | |
| 81 | H | 0 | 2-Cl-5-$CH_3$ | $CH_3$ | $OCH_3$ | | |
| 82 | H | 0 | 2,3,6-$(CH_3)_3$ | $CH_3$ | $OCH_3$ | | |
| 83 | H | 0 | 2,6-$(NO_2)_2$ | $CH_3$ | $OCH_3$ | | |
| 84 | H | 0 | 3-$C_2H_5$ | $CH_3$ | $OCH_3$ | | |
| 85 | H | 0 | 3-$CH(CH_3)_2$ | $CH_3$ | $OCH_3$ | | |
| 86 | H | 0 | 2,6-$Br_2$-3,5$(CH_3)_2$ | $CH_3$ | $OCH_3$ | | |

Tabelle : Fortsetzung

| Beispiel Nr. | X | m | $(R^1)_n$ | $R^2$ | $R^3$ | Schmp. °C; Sdp. °C/mbar | $[\alpha]_D^{20}$ $n_D^{20}$; Wellenlänge einer Bande im IR-Spektrum |
|---|---|---|---|---|---|---|---|
| 87 | H | 0 | $2-NO_2-6C(CH_3)_3$ | $CH_3$ | $OCH_3$ | | |
| 88 | H | 0 | $2-NO_2-3CH_3-6Cl$ | $CH_3$ | $OCH_3$ | | |
| 89 | H | 0 | $2-(CH_3O)-6-NO_2$ | $CH_3$ | $OCH_3$ | | |
| 90 | H | 2 | 2-Cl | $C_2H_5$ | $OCH_3$ | 85- 88 | |
| 91 | Cl | 2 | 2-Cl | $CH_3$ | $OCH_3$ | | $[\alpha]_D^{25}$ = + 28.7 |
| 92 | Cl | 1 | 2-Cl | $CH_3$ | $OCH_3$ | | $[\alpha]_D^{25}$ = + 30.1 |
| 93 | Cl | 0 | 2-Cl | $CH_3$ | $OCH_3$ | | $[\alpha]_D^{20}$ = + 29.2 |
| 94 | H | 0 | H | $C_2H_5$ | $OCH_3$ | | 1.5408 |
| 95 | H | 0 | $2-CH_3$ | $CH_3$ | OH | 99-102 | |
| 96 | H | 0 | $2-CH_3$ | $CH_3$ | $ONH_2(CH_3)_2$ | | 1.5517 |
| 97 | H | 0 | $2-CH_3$ | $CH_3$ | $ONH_3CH_2CH(CH_3)_2$ | 109-112 | |

Die neuen Verbindungen können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z. B. Sonnenschein, Dauer, Durchschnittstemperatur, Niederschlagsmenge,
e) von der Bodenbeschaffenheit (einschließlich Düngung),
f) von der Formulierung bzw. Anwendungsform des Wirkstoffs und schließlich,
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf ; außerdem ist eine dunklere Blattfärbung zu beobachten.

Als vorteilhaft für die Praxis erweist sich z. B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des « Lagerns » (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.

Mit Wachstumshemmern läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z. B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und — wegen der relativ geringen Blatt- bzw. Pflanzenmasse — dem Befall mit verschiedenen Krankheiten (z. B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den neuen Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bie anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sproßteil der

Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z. B. Getreide wie Weizen, Gerste, Roggen, Hafer und Reis oder Mais oder Gräsern als auch insbesondere bei Dikotylen (z. B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemäßen Wirkstoffe können den Kulturpflanzen sowohl vom Samen her (als Saatgutbeizmittel) als auch über den Boden, d. h. durch die Wurzel sowie — besonders bevorzugt — durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha bevorzugt 0,25 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin), Dimethylformamid und Wasser ; feste Trägerstoffe wie natürliche Gesteinsmehle (z. B. Kaoline, Tonerden, Talkum, Kreide) und synthetische Gesteinsmehle (z. B. hochsdisperse Kieselsäure, Silikate) ; Emulgiermittel oder sonstige oberflächenaktive Mittel, wie nichtionogene und anionische Emulgatoren (z. B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

## Wirkungsbeispiele

a) Gewächshausversuche

Zur Bestimmung der wachstumsregulierenden Eigenschaft der erfindungsgemäßen Wirkstoffe wurden Testpflanzen auf ausreichend mit Nährstoffen versorgtem Kultursubstrat in Kunststoffgefäßen von ca. 12,5 cm Durchmesser angezogen. Im Nachauflaufverfahren wurden die zu prüfenden Substanzen in wäßriger Aufbereitung auf die Pflanzen gesprüht.

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente Chlorcholinchlorid (CCC).

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Monokotyle
Sommergerste, Sorte « Union »
Nachauflaufverfahren

| Nr. d. chem. Beispiele | Konz. mg WS/Gef. | Wuchshöhen rel. |
|---|---|---|
| unbeh. | - | 100 |
| CCC | 1,5 | 92,8 |
| | 6 | 91,2 |
| 6 | 1,5 | 58,2 |
| | 6 | 50,9 |

Fortsetzung

| Nr. d. chem. Beispiele | Konz. mg WS/Gef. | Wuchshöhen rel. |
|---|---|---|
| 66 | 1,5 | 87,6 |
| | 6 | 77,7 |
| 1 | 1,5 | 87,0 |
| | 6 | 72,8 |
| 31 | 1,5 | 83,1 |
| | 6 | 74,9 |
| 7 | 1,5 | 70,0 |
| | 6 | 52,1 |

Dikotyle
Sonnenblumen, Sorte « Sorex »
Nachauflaufverfahren

| Nr. d. chem. Beispiele | Konz. mg WS/Gef. | Wuchshöhen rel. |
|---|---|---|
| unbeh. | - | 100 |
| CCC | 1,5 | 91,3 |
| | 6 | 83,7 |
| 6 | 1,5 | 68,4 |
| | 6 | 64,6 |
| 31 | 1,5 | 81,1 |
| | 6 | 70,1 |
| 7 | 1,5 | 71,9 |
| | 6 | 55,3 |
| 8 | 1,5 | 62,7 |
| | 6 | 55,3 |
| 37 | 1,5 | 67,1 |
| | 6 | 63,6 |
| 93 | 1,5 | 64,6 |
| | 6 | 62,7 |
| 95 | 1,5 | 79,8 |
| | 6 | 72,2 |
| 96 | 1,5 | 68,4 |
| | 6 | 60,8 |
| 92 | 1,5 | 65,7 |
| | 6 | 54,7 |
| 91 | 1,5 | 62,0 |
| | 6 | 54,7 |

Sojabohnen, Sorte « Gieso »
Nachauflaufverfahren

| Nr. d. chem. Beispiele | Konz. mg WS/Gef. | Wuchshöhen rel. |
|---|---|---|
| unbeh. | - | 100 |
| CCC | 0,5 | 94,1 |
| | 1,5 | 94,1 |
| 6 | 0,5 | 70,9 |
| | 1,5 | 67,2 |
| 2 | 0,5 | 56,9 |
| | 1,5 | 55,3 |
| 7 | 0,5 | 76,9 |
| | 1,5 | 66,8 |
| 8 | 0,5 | 72,9 |
| | 1,5 | 72,9 |
| 93 | 0,5 | 77,1 |
| | 1,5 | 67,5 |
| 95 | 0,5 | 84,3 |
| | 1,5 | 74,7 |
| 96 | 0,5 | 81,9 |
| | 1,5 | 79,5 |

b) Vegetationsversuche

Vegetationsversuch zu Sommergerste

In einem Vegetationsversuch in Mitscherlich-Gefäßen wurde Sommergerste der Sorte « Aramir » auf einem neutralen, lehmigen Sandboden angezogen. Die Düngung erfolgte mit 1,5 g N als Ammonnitrat und 1,0 g $P_2O_5$ als sekundäres Kaliumphosphat zur Aussaat. Die Wirkstoffe wurden in wäßriger Aufbereitung mit Aufwandmengen von 10 und 20 mg WS/Gef. zum Stadium 30 (Zadoks) auf die Pflanzen gesprüht.

Als Vergleichssubstanz diente Chlorcholinchlorid (CCC).

Bei Versuchsende zeigten die behandelten Pflanzen gegenüber der Kontrolle ein stark verringertes Längenwachstum.

| Nr. d. chem. Beispiels | Konz. mg WS/Gef. | Halmlänge cm | Halmlänge rel. |
|---|---|---|---|
| unbeh. | - | 74,1 | 100 |
| CCC | 10 | 72,8 | 98,3 |
| | 20 | 72,0 | 97,2 |
| 6 | 10 | 52,9 | 71,4 |
| | 20 | 49,9 | 67,3 |

Vegetationsversuch zu Sojabohnen

Unter den gleichen Bedingungen wie im vorstehenden Versuch wurden Sojabohnen der Sorte

« Gieso » angezogen. Die Düngung erfolgte mit 0,5 g N als Ammonnitrat und 0,5 g $P_2O_5$ als sekundäres Kaliumphosphat zur Saat. Die Wirkstoffe wurden in wäßriger Aufbereitung bei einer Wuchshöhe von 40 cm auf die Pflanzen gesprüht. Bei Versuchsende zeigten die behandelten Pflanzen gegenüber der Kontrolle ein verringertes Längenwachstum.

| Nr. d. chem. Beispiels | Konz. mg WS/Gef. | Halmlänge cm | rel. |
|---|---|---|---|
| unbeh. | - | 61,1 | 100 |
| 6 | 2,5 | 47,5 | 77,7 |
| | 5,0 | 45,8 | 75,0 |

Vegetationsversuch zu Erdnüssen

In einem Vegetationsversuch wurden auf einem neutralen, lehmigen Sandboden Erdnüsse angezogen. Die Düngung erfolgte mit 1,5 g N als Ammonnitrat und 1,0 g $P_2O_5$ als sekundäres Kaliumphosphat zur Aussaat. Der Wirkstoff wurde mit Aufwandmengen von 0,75 und 1,5 kg/ha bei einer Pflanzenhöhe von 15 bis 18 cm zur Blüte über das Blatt in üblicher Weise appliziert.

Nach der Ernte zeigte sich bei den behandelten Pflanzen sowohl bei der Anzahl der Nüsse als auch beim Frischgewicht ein deutlicher Mehrertrag.

| Nr. d. chem. Beispiels | Konz. mg WS/Gef. | Zahl d. Nüsse /Gef. rel. | FS/Gef. rel. |
|---|---|---|---|
| unbeh. | - | 100 | 100 |
| 6 | 2,5 | 132 | 132 |
| | 5 | 155 | 142 |
| 22 | 2,5 | 128 | 135 |
| | 5 | 115 | 122 |

Vegetationsversuch zu Erdnüssen

In einem Vegetationsversuch wurden auf einem neutralen, lehmigen Sandboden Erdnüsse angezogen. Die Düngung erfolgte mit 1,5 g N als Ammonnitrat und 1,0 g $P_2O_5$ als sekundäres Kaliumphosphat zur Aussaat. Der Wirkstoff wurde mit Aufwandmengen von 0,75 und 1,5 kg/ha bei einer Pflanzenhöhe von 15 bis 18 cm zur Blüte über das Blatt in üblicher Weise appliziert.

Nach der Ernte zeigte sich bei den behandelten Pflanzen sowohl bei der Anzahl der Nüsse als auch beim Frischgewicht ein deutlicher Mehrertrag.

| Nr. d. chem. Beispiels | Konz. mg WS/Gef. | Zahl d. Nüsse /Gef. abs. | rel. | FS/Gef. abs. | rel. |
|---|---|---|---|---|---|
| unbeh. | - | 53 | 100 | 43,1 | 100 |
| 22 | 2,5 | 68 | 128 | 58,2 | 135 |
| | 5 | 61 | 115 | 52,7 | 122 |

**Patentansprüche**

1. Phenoxyalkansäureverbindungen der allgemeinen Formel I

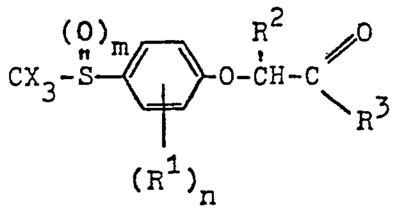

(I)

worin

$R^1$ Wasserstoff, Halogen, $C_1$-$C_4$-alkyl, $C_1$-$C_5$-alkoxy, Nitro, Cyano, Trifluormethyl,
$R^2$ Wasserstoff, $C_1$-$C_3$-alkyl,
$R^3$ die Gruppen OM, $OR^4$,

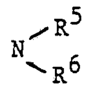

bedeutet,
worin

M für ein Äquivalent eines Metallions oder ein gegebenenfalls substituiertes Ammoniumion,

$R^4$ für einen $C_1$-$C_{12}$-Alkylrest, der gegebenenfalls durch eine oder mehrere Hydroxy-, Alkoxy-, Aminogruppen oder ein oder mehrere Halogenatome substituiert sein kann, einen gegebenenfalls in gleicher Weise substituierten Alkenyl-, Alkinyl-, Cycloalkyl-, Cycloalkenyl- oder Arylalkylrest,

$R^5$ für Wasserstoff oder für die von $R^4$ bezeichneten Reste und

$R^6$ für die von $R^5$ bezeichneten Reste und eine $C_1$-$C_6$-Alkoxygruppe und eine $C_1$-$C_6$-Alkylamino- oder Dialkylaminogruppe steht, und außerdem $R^5$ und $R^6$ zusammen mit dem benachbarten Stickstoff ein gegebenenfalls substituiertes heterocyclisches Ringsystem bilden können,
und

jedes X unabhängig voneinander Halogen oder Wasserstoff,
m gleich Null, 1 oder 2, und
n gleich Null, 1, 2, 3 oder 4 ist.

2. Verfahren zur Herstellung der Verbindungen gemäß Anspruch 1, dadurch gekennzeichnet, daß man entweder eine Verbindung der Formel II

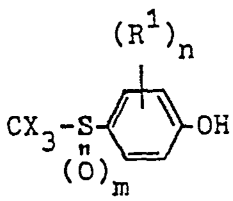

(II)

worin $R^1$, X, m und n die oben angegebenen Bedeutungen besitzen, oder deren Salze mit einer Verbindung der Formel III

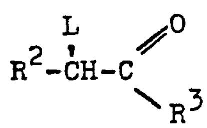

(III)

worin $R^2$ und $R^3$ die oben angegebenen Bedeutungen haben und L einen nucleophil verdrängbaren Substituenten bedeutet,
gegebenenfalls in Gegenwart eines Verdünnungsmittels und/oder einer anorganischen oder organischen Base bei Temperaturen zwischen 10 °C und dem Siedepunkt des Gemisches, gegebenenfalls in Gegenwart eines Reaktionsbeschleunigers umsetzt, oder

ein Alkylthiophenol der Formel IV

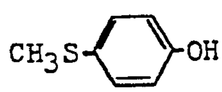

(IV)

mit einer Verbindung der Formel III zu einer Verbindung der Formel V

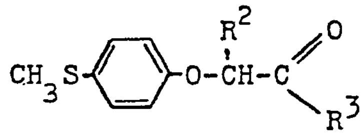

(V)

worin $R^2$ und $R^3$ die oben angegebenen Bedeutungen haben, gegebenenfalls in Gegenwart eines Verdünnungsmittels und/oder einer anorganischen oder organischen Base bei Temperaturen zwischen 20 °C und dem Siedepunkt des Gemisches gegebenenfalls in Gegenwart eines Reaktionsbeschleunigers umsetzt und diese mit einem Halogenierungsmittel in Verbindung der Formel VI

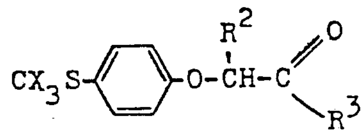

(VI)

worin X, $R^2$ und $R^3$ die oben genannten Bedeutungen haben, gegebenenfalls in Gegenwart eines Verdünnungsmittels bei Temperaturen zwischen 0 °C und dem Siedepunkt des Gemisches, gegebenenfalls in Gegenwart eines Reaktionsbeschleunigers überführt.

3. Mittel zur Beeinflussung des Pflanzenwachstums, enthaltend mindestens eine Verbindung gemäß Anspruch 1.

4. Mittel zur Beeinflussung des Pflanzenwachstums, enthaltend mindestens eine Verbindung gemäß Anspruch 1 und einen festen oder flüssigen Trägerstoff.

5. Verfahren zur Beeinflussung des Pflanzenwachstums, dadurch gekennzeichnet, daß man mindestens eine Phenoxyalkansäure-Verbindung gemäß Anspruch 1 auf die Pflanze einwirken läßt.

6. Verfahren zur Herstellung von Mitteln zur Beeinflussung des Pflanzenwachstums, dadurch gekennzeichnet, daß man Phenoxyalkansäureverbindungen der Formel I gemäß Anspruch 1 mit festen oder flüssigen Trägerstoffen mischt.

7. 2-[2'-Chlor-4'-trifluormethylsulfinyl]-phenoxypropionsäuremethylester.

8. 2-[2'-Chlor-4'-trifluormethylthio]-phenoxypropionsäuremethylester.

9. 2-[2'-Chlor-4'-trifluormethylsulfonyl]-phenoxypropionsäuremethylester.

10. 2-[2'-Chlor-4'-difluormethylthio]-phenoxypropionsäuremethylester.

11. 2-[2'-Chlor-4'-trichlormethylthio]-phenoxypropionsäuremethylester.

**Claims**

1. A phenoxyalkanoic acid compound of the formula I

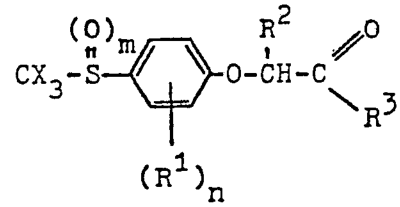

(I)

where

$R^1$ is hydrogen, halogen, $C_1$-$C_4$-alkyl, $C_1$-$C_5$-alkoxy, nitro, cyano or trifluoromethyl,

$R^2$ is hydrogen or $C_1$-$C_3$-alkyl,

$R^3$ is OM, $OR^4$ or

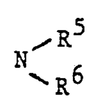

where

M is one equivalent of a metal ion or an unsubstituted or substituted ammonium ion,

$R^4$ is $C_1$-$C_{12}$-alkyl which can be unsubstituted or substituted by one or more hydroxyl, alkoxy or amino groups or by one or more halogen atoms, or is an unsubstituted or similarly substituted alkenyl, alkynyl, cycloalkyl, cycloalkenyl or aralkyl radical,

$R^5$ is hydrogen or one of the radicals mentioned for $R^4$, and

$R^6$ is one of the radicals mentioned for $R^5$ or is $C_1$-$C_6$-alkoxy, $C_1$-$C_6$-alkylamino or dialkylamino, and furthermore $R^5$ and $R^6$, together with the adjacent nitrogen atom, can form an unsubstituted or substituted heterocyclic ring system,

and

the radicals X independently of one another are halogen or hydrogen,

m is 0, 1 or 2, and

n is 0, 1, 2, 3 or 4.

2. A process for the manufacture of a compound as claimed in claim 1, wherein either a compound of the formula II

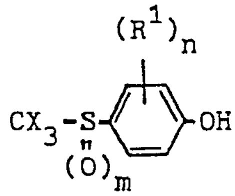

(II)

where $R^1$, X, m and n have the above meanings, or one of its salts, is reacted with a compound of the formula III

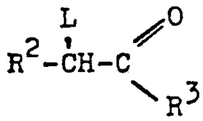

(III)

where $R^2$ and $R^3$ have the above meanings and L is a substituent which can undergo nucleophilic displacement, in the presence or absence of a diluent and/or of an inorganic base, at from 10 °C to the boiling point of the mixture, and in the presence or absence of a reaction accelerator, or an alkylthiophenol of the formula IV

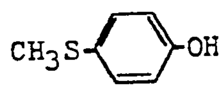

(IV)

is reacted with a compound of the formula III, in the presence or absence of a diluent and/or of an inorganic or organic base, at from 20 °C to the boiling point of the mixture and in the presence or absence of a reaction accelerator, to give a compound of the formula V

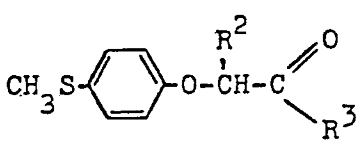

(V)

where $R^2$ and $R^3$ have the above meanings, and the product is converted with a halogenating agent, in the presence or absence of a diluent, at from 0 °C to the boiling point of the mixture and in the presence or absence of a reaction accelerator, into a compound of the formula VI

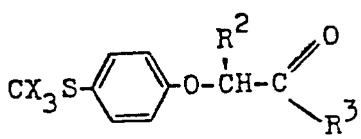

(VI)

where X, $R^2$ and $R^3$ have the above meanings.

3. An agent for influencing plant growth, containing at least one compound as claimed in claim 1.

4. An agent for influencing plant growth, containing at least one compound as claimed in claim 1 and a solid or liquid carrier.

5. A process for influencing plant growth, wherein at least one phenoxyalkanoic acid compound as claimed in claim 1 is allowed to act on the plants.

6. A process for the preparation of agents for influencing plant growth, wherein phenoxyalkanoic acid compounds of the formula I as claimed in claim 1 are mixed with solid or liquid carriers.

7. Methyl 2-[2'-chloro-4'-trifluoromethylsulfinylphenoxy]-propionate.

8. Methyl 2-[2'-chloro-4'-trifluoromethylthiophenoxy]-propionate.

9. Methyl 2-[2'-chloro-4'-trifluoromethylsulfonylphenoxy]-propionate.

10. Methyl 2-[2'-chloro-4'-difluoromethylthiophenoxy]-propionate.

11. Methyl 2-[2'-chloro-4'-trichloromethylthiophenoxy]-propionate.

**Revendications**

1. Dérivés d'acides phénoxy-alcanoïques de la formule générale I

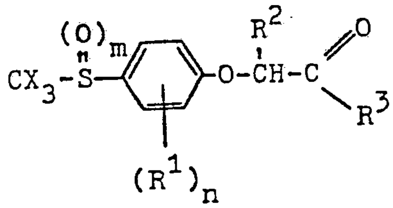

(I)

dans laquelle

$R^1$ désigne un atome d'hydrogène, un radical alkyle en $C_1$ à $C_4$ ou alcoxy en $C_1$ à $C_5$ ou un groupe nitro, cyano ou trifluorométhyle,

$R^2$ désigne un atome d'hydrogène ou un radical alkyle en $C_1$ à $C_3$,

$R^3$ = OM, $OR^4$ ou

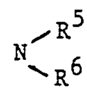

où

M représente un équivalent d'un ion de métal ou un ion ammonium éventuellement substitué,

$R^4$ désigne un radical alkyle en $C_1$ à $C_{12}$, éventuellement substitué par un ou plusieurs atomes d'halogène ou un ou plusieurs groupes hydroxy, alcoxy ou amino, ou un groupe alcényle, alcynyle, cycloalkyle, cycloalcényle ou arylalkyle pouvant être substitué de façon analogue,

$R^5$ désigne un atome d'hydrogène ou possède les significations définies pour $R^4$,

$R^6$ possède les significations définies pour $R^5$ ou représente un groupe alcoxy en $C_1$ à $C_6$ ou alkyl (en $C_1$ à $C_6$)-amino ou dialkyl-amino, $R^5$ et $R^6$ pouvant en outre constituer avec l'atome d'azote adjacent un système hétérocyclique éventuellement substitué ; chaque

X représente, indépendamment des autres, un atome d'hydrogène ou d'halogène,

m vaut 0, 1 ou 2 et

n vaut 0, 1, 2, 3 ou 4.

2. Procédé de préparation de composés selon la revendication 1, caractérisé en ce que l'on fait réagir soit un composé de la formule II

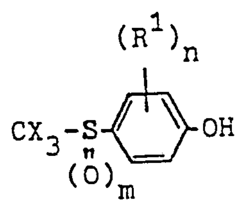

(II)

dans laquelle $R^1$, X, m et n possèdent les significations définies ci-dessus, ou un de ses sels, à des températures comprises entre 10 °C et le point d'ébullition du mélange réactionnel, éventuellement dans un diluant et(ou) en présence d'une base organique ou inorganique et le cas échéant en présence d'un accélérateur de la réaction, avec un composé de la formule III

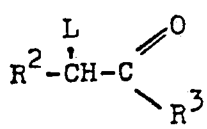

(III)

dans laquelle $R^2$ et $R^3$ possèdent les significations définies plus haut et L désigne un substituant clivable par voie nucléophile, soit un alkyl-thiophénol de la formule IV

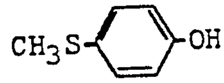

(IV)

à des températures comprises entre 20 °C et le point d'ébullition du mélange réactionnel, éventuellement dans un diluant et(ou) en présence d'une base organique ou inorganique et le cas échéant en présence d'un accélérateur de la réaction, avec un composé de la formule III, puis le composé obtenu de la formule V

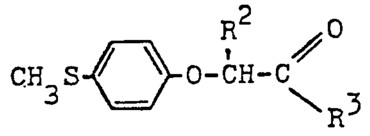

(V)

dans laquelle $R^2$ et $R^3$ possèdent les significations définies, à des températures comprises entre 0 °C et le point d'ébullition du mélange réactionnel, éventuellement dans un diluant et(ou) en présence d'un accélérateur de la réaction, avec un agent halogénant pour obtenir un composé de la formule VI

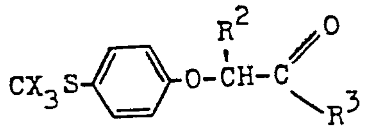

(VI)

dans laquelle X, $R^2$ et $R^3$ possèdent les significations définies précédemment.

3. Composition destinée à influencer la croissance de plantes, contenant au moins un composé selon la revendication 1.

4. Composition destinée à influencer la croissance de plantes, contenant au moins un dérivé selon la revendication 1 et une matière support solide ou un véhicule liquide.

5. Procédé pour influencer la croissance de plantes, caractérisé en ce que l'on laisse agir sur les plantes au moins un dérivé d'acide phénoxy-alcanoïque selon la revendication 1.

6. Procédé de préparation de compositions destinées à influencer la croissance de plantes, caractérisé en ce que l'on mélange des dérivés d'acides phénoxy-alcanoïques de la formule I selon la revendication 1 à des matières supports solides ou des véhicules liquides.

7. Le 2-(2'-chloro-4'-trifluorométhyl-sulfinyl)-phénoxy-propionate de méthyle.

8. Le 2-(2'-chloro-4'-trifluorométhyl-thio)-phénoxy-propionate de méthyle.

9. Le 2-(2'-chloro-4'-trifluorométhyl-sulfonyl)-phénoxy-propionate de méthyle.

10. Le 2-(2'-chloro-4'-difluorométhyl-thio)-phénoxy-propionate de méthyle.

11. Le 2-(2'-chloro-4'-trichlorométhyl-thio)-phénoxy-propionate de méthyle.